(19) Europäisches Patentamt

European Patent Office

Office européen des brevets

(11) Veröffentlichungsnummer: **0 670 159 A1**

## (12) EUROPÄISCHE PATENTANMELDUNG

(21) Anmeldenummer: **95102244.1**

(22) Anmeldetag: **18.02.95**

(51) Int. Cl.⁶: **A61K 9/00**

(30) Priorität: **22.02.94 DE 4405627**

(43) Veröffentlichungstag der Anmeldung:
**06.09.95 Patentblatt 95/36**

(84) Benannte Vertragsstaaten:
**CH DE FR GB IT LI NL**

(71) Anmelder: **HOECHST AKTIENGESELLSCHAFT**

**D-65926 Frankfurt am Main (DE)**

(72) Erfinder: **von Werner, Konrad, Dr.**
**Wehrstrasse 6**
**D-84518 Garching (DE)**
Erfinder: **Gross, Udo, Dr.**
**Falkenberger Chaussee 91**
**D-13059 Berlin (DE)**

(54) **Fluorkohlenwasserstoffe enthaltende Ölemulsionen.**

(57) Emulgatorhaltige wäßrige Emulsionen von Ölen, die eine Verbindung der Formel I

$(R_F)_x\text{-}R_H$ (I)

enthalten, in der $R_F$ einen hochfluorierten Alkylrest bedeutet, der unmittelbar oder über ein Bindeglied an die Gruppe $R_H$ gebunden ist, die für einen Alkanrest oder Wasserstoff steht, und x eine Zahl von 1 bis 4 bedeutet, eignen sich als Träger für Gase und Wirkstoffe.

EP 0 670 159 A1

Rank Xerox (UK) Business Services
(3.10/3.09/3.3.4)

Die Erfindung betrifft emulgatorhaltige wäßrige Emulsionen von Ölen, die zusätzlich eine Verbindung der Formel I

$$(R_F)_x\text{-}R_H \qquad (I)$$

enthalten. In dieser Formel bezeichnet $R_F$ einen hochfluorierten, vorzugsweise perfluorierten Alkylrest, der unmittelbar oder über ein Bindeglied an die Gruppe $R_H$ gebunden ist, die für einen Alkanrest oder Wasserstoff steht, und x steht für eine Zahl von 1 bis 4. Das Bindeglied ist ein Sauerstoff- oder Schwefelatom oder eine Gruppe der Formel II

$$\text{-}(O\text{-}CH_2\text{-}CH_2)_p\text{-} \qquad (II),$$

in der p für 1 oder 2 steht, mit der Maßgabe, daß $R_H$ nicht Wasserstoff bedeutet, wenn $R_F$ über Sauerstoff oder Schwefel gebunden ist.

Der Alkanrest $R_H$ kann - außer den Resten $R_F$ - weitere Substituenten tragen, beispielsweise Cycloalkylreste wie Cyclohexyl oder Dioxanylreste, vorzugsweise steht er für einen (unsubstituierten) Rest der Formel III

$$C_qH_{2q+1-x} \qquad (III)$$

in der x die obengenannte Bedeutung hat und q für eine ganze Zahl von 1 bis 12 steht.

In einer bevorzugten Ausgestaltung der Erfindung entspricht die Verbindung der Formel I einer Verbindung der Formel IV

$$C_nF_{2n+1}\text{-}X\text{-}C_mH_{2m+1} \qquad (IV).$$

In dieser Formel bedeutet n 1 bis 20, wobei der Rest $C_nF_{2n+1}$ endverzweigt oder vorzugsweise linear ist, X entspricht dem vorstehend genannten Bindeglied und m entspricht einer Zahl von 0 bis 12, wenn X eine Gruppe der Formel II ist, oder einer Zahl von 1 bis 12, wenn X Sauerstoff oder Schwefel ist, wobei der Rest $C_mH_{2m+1}$ vorzugsweise linear ist.

In einer weiteren bevorzugten Ausgestaltung der Erfindung entspricht die Verbindung der Formel I einem Oligomer einer Verbindung V

$$Y\text{-}(CF_2)_a\text{-}O_b\text{-}(CH_2)_c\text{-}CH=CH_2 \qquad (V)$$

sowie einem Co-Oligomer aus einer Verbindung der Formel V mit Verbindungen der Formel VI

$$Y\text{-}(CX_2)_d\text{-}O_b\text{-}(CX_2)_c\text{-}CX=CX_2 \qquad (VI)$$

in denen Y Wasserstoff oder Fluor, a eine Zahl von 2 bis 16, b und c - unabhängig voneinander - 0 oder 1 und d eine Zahl von 0 bis 6 bedeuten, mit einem mittleren Oligomerisationsgrad von 2 bis 4.

Bevorzugt sind Oligomere von Verbindungen der Formel V, in denen Y Fluor, a eine Zahl von 4 bis 12 und b und c Null bedeuten.

Die genannten Oligomeren und Co-Oligomeren sind aus der EP-A 545 174 bekannt.

Als Öle werden nicht mit Wasser mischbare, bei Zimmertemperatur flüssige Verbindungen aus der Reihe der fetten Öle (Triglyceride) und der Kohlenwasserstofföle, wie beispielsweise Paraffinöle, eingesetzt.

Die Wahl des Emulgators richtet sich nach der Art der Emulsion, die vom Typ Wasser-in-Öl oder Öl-in-Wasser sein kann, der Art der emulgierten Substanzen und des beabsichtigten Einsatzzweckes. Soll die erfindungsgemäße Emulsion beispielsweise im medizinischen oder kosmetischen Bereich Verwendung finden, so muß selbstverständlich ein physiologisch verträglicher Emulgator herangezogen werden. Der Fachmann wird somit Art und Menge des Emulgators nach seinem Fachwissen und gegebenenfalls anhand einfacher Vorversuche auswählen.

Emulgatorhaltige wäßrige Emulsionen von Ölen, die zusätzlich eine hochfluorierte organische Verbindung enthalten, sind aus der EP-A 231 091 bekannt. Als hochfluorierte organische Verbindungen werden allgemein auch teilweise fluorierte Kohlenwasserstoffe genannt, als spezifische Verbindungen jedoch nur Perfluor-5,6-dihydro-5-decen und Perfluor-4,5-dihydro-4-octen aufgeführt. In diesen beiden Verbindungen ist also das Kohlenwasserstoffsegment mittelständig und ungesättigt. Es wurde aber gefunden, daß diese Verbindungen den Nachteil aufweisen, daß sie schwieriger in Öl-in-Wasser- oder Wasser-in-Öl-Emulsionen einzubringen sind als typische Verbindungen der Formel I. Ein weiterer Nachteil dieser internen Alkene besteht darin, daß ihre Synthese aufwendig ist und sie daher nicht im technischen Maßstab verfügbar sind.

Aus WO 93/01798 sind emulgatorhaltige wäßrige Emulsionen von fluorierten Kohlenwasserstoffen bekannt, die eine untergeordnete Menge an einer lipophilen-fluorophilen Verbindung mit einer fluorierten Region und einer Kohlenwasserstoffregion als Stabilisator enthalten. Wesentlich für die Stabilität der Zubereitung ist das Zusammenspiel des hydrophilen-lipophilen Emulgators und der lipophilen- fluorophilen Verbindung, die in Wechselwirkung mit der zu emulgierenden fluorierten Kohlenstoffverbindung tritt. Sowohl die zu emulgierende Verbindung als auch der Stabilisator können Verbindungen der Formel I sein. Überraschenderweise wurde jedoch gefunden, daß die fluorophile-lipophile Verbindung in Kombination mit den erfindungsgemäß eingesetzten Ölen sehr stabile Emulsionen ergibt, und zwar auch dann, wenn die Verbindung der Formel I nicht nur in untergeordneten Mengen, sondern - bezogen auf die nichtwäßrigen Anteile der Emulsion - als Hauptkomponente vorliegt.

Die Proportionen der Bestandteile in den erfindungsgemäßen Emulsionen können in weiten Grenzen schwanken. Der Anteil des Wassers richtet sich nach dem Typ der Emulsion (Wasser-in-Öl oder Öl-in-Wasser) wie auch die Menge des Emulgators. Von den emulgierten Stoffen liegt üblicherweise die Verbindung der Formel I, bezogen auf das Gewicht, im Überschuß vor. Bevorzugte Emulsionen enthalten 5 bis 45 Gew.-%, insbesondere 10 bis 40 Gew.-%, an Öl, bezogen auf die Summe von Öl und Verbindung der Formel I.

Die Herstellung der erfindungsgemäßen Emulsionen erfolgt durch Mischen der Verbindung der Formel I mit dem Öl, worauf dieser Mischung das Wasser und der Emulgator zugesetzt werden. Die Art des Mischens und des Zusetzens richtet sich nach Art und Menge der Komponenten, wobei der Fachmann die zweckmäßigste Technik anhand seines Fachwissens und gegebenenfalls anhand einfacher Vorversuche ermittelt. So kann die wäßrige Emulgatorlösung oder -emulsion dem Gemisch absatzweise oder kontinuierlich zugesetzt werden, wobei für die entsprechende Durchmischung gesorgt wird.

Nötigenfalls wird die so erhaltene Zubereitung anschließend noch homogenisiert. Diese Homogenisierung kann in einem Hochdruckhomogenisator bei etwa 100 bis 600 bar, vorzugsweise bei 200 bis 600 bar, insbesondere bei etwa 300 bar, erfolgen. Geeignet sind auch Ultraschall-Desintegratoren oder Mikrofluidizer. Man kann so erfindungsgemäß stabile Emulsionen mit einer mittleren Teilchengröße der emulgierten Stoffe von 80 bis 120 nm, insbesondere 90 bis 110 nm, erhalten. Diese Tröpfchengröße und die enge Partikelgrößenverteilung bleiben auch nach einer Sterilisierung (im Autoklav bei 121 °C) im wesentlichen erhalten. Demgegenüber werden in der WO 93/01798 nach der Sterilisierung Teilchengrößen von 130 bis 400 nm angegeben.

Die erfindungsgemäßen Emulsionen eignen sich zum Transport von Sauerstoff in chemischen und vorzugsweise biologischen beziehungsweise medizinischen Systemen, beispielsweise im vaskulären System oder in der Dermis, und in der Biotechnik bei der Fermentation und Zellzüchtung. Weiterhin eignen sich die Emulsionen als Carrier von Wirkstoffen und Kontrastmitteln und als Standard in der Blutgaskontrolle.

Je nach dem gewünschten Einsatzzweck wird also die Sauerstofftransportkapazität, die Viskosität, die Lipidlöslichkeit, die Teilchengröße oder das Einschlußvermögen von Wirkstoffen in micellare oder vesikuläre Aggregate oder eine Kombination dieser Eigenschaften im Vordergrund stehen. Der Fachmann wird auch hier aufgrund seines Fachwissens und gegebenenfalls einfacher Vorversuche die Art und Menge der Komponenten auswählen. Lediglich beispielsweise sei auf die Auswahl der Emulgatoren hingewiesen: Für medizinische Zwecke werden nichttoxische Emulgatoren verwendet, beispielsweise die nicht hämatolytisch wirkenden Phospholipide wie Phosphatidylcholin oder die Ethylenoxid-Propylenoxid-Blockpolymeren, die in großer Auswahl im Handel zur Verfügung stehen. In topischen Systemen wie Hautcremes, wo der Vorteil einer sauerstoffdurchlässigen Barriere, die die Haut vor chemischen und anderen Noxen schützt, zum Tragen kommt, eignen sich hautfreundliche Emulgatoren wie Laurylethersulfate, Laurylsulfosuccinate, Cetylphosphate, Alkylpolyoxyethylen-tridecylethercarboxylate, Fettsäureamidopropylbetaine, Amphocarboxyglycinate und analoge Verbindungen.

Die Verbindungen der Formel I sind dermatologisch unbedenklich, weshalb sich daraus hergestellte erfindungsgemäße Emulsionen besonders für topische Anwendungen eignen. Hierzu werden die Emulsionen mit Hilfe üblicher Zusätze in geeignete Salben, Gele oder Lotionen eingearbeitet. Mit Hilfe dieser Applikationsformen ist eine zusätzliche Sauerstoffversorgung auf und in der Haut möglich. So wird der Heilprozeß von Hautverletzungen und Verbrennungen beschleunigt, da der notwendige Sauerstoff für die Collagenbildung durch Hydroxylierung von Prolin zur Verfügung gestellt wird. Neben seiner Funktion als Gasträger können die erfindungsgemäßen Emulsionen auch als Carrier für Wirkstoffe fungieren, die über die Haut aufgenommen werden. Die guten Lösungseigenschaften der erfindungsgemäßen Emulsionen erlauben die Inkorporation von Wirkstoffen, die aus einem sehr breiten Wirkbereich ausgewählt sein können.

So kann unter anderem ein Diagnostikum einer erfindungsgemäßen Emulsion zugefügt und topisch appliziert werden. So können beispielsweise persistente Fluorcarbonradikale wie $.C_{10}F_{21}$ in eine erfindungsgemäße Emulsion eingearbeitet werden und auf die menschliche Haut aufgetragen werden. Die Penetrationstiefe und -geschwindigkeit dieser Radikale lassen sich ESR-spektroskopisch nach der Methode des Dermascanning bestimmen. Als Penetrationspromotor können der Zubereitung N-Alkylaminobenzoate zugesetzt werden.

Als Hautcreme, die einen gasdurchlässigen Schutzfilm auf der Haut auszubilden vermag, dient beispielsweise eine erfindungsgemäße Emulsion mit einer Verbindung der Formel V, Paraffinöl und einem Phospholipid als Emulgator. Man erhält so eine "Barriere"-Creme, die als "chemischer Handschuh" auf der Haut einen Film gegen die schädigende Wirkung von Chemikalien, Lösemitteln, Ölen, Farben oder Metallpulvern ausbildet. Die Creme kann weiterhin zur Behandlung von chronischer Dermatitis der Hände dienen.

Neben diesen topischen Applikationen können die erfindungsgemäßen Emulsionen auf allen Gebieten eingesetzt werden, auf denen die bekannten Emulsionen von Perfluorcarbonverbindungen Verwendung finden. Der Vorteil der Verbindungen der Formel I ist hierbei auch darin zu sehen, daß diese Verbindungen außerordentlich stabil sind und auch im Organismus nicht abgebaut und metabolisiert werden.

Die erfindungsgemäßen Emulsionen sind sehr stabil. Maßgeblich hierfür ist die mehr oder weniger große Mischbarkeit der Verbindung der Formel I mit dem Öl unter der Mitwirkung des Emulgators. Es wurde gefunden, daß als Kriterium die kritische Löslichkeitstemperatur (CST) der Verbindung der Formel I in n-Hexan dienen kann. Diese Größe entspricht der Lipidlöslichkeit und diese korreliert mit der Respiration und Exhalation aus dem Gewebe. Die Lipidlöslichkeit entspricht der Zellmembranlöslichkeit, so daß der CST-Wert auch eine Kenngröße für die Verweilzeit in Organen, beispielsweise nach einer Infusion in der Leber oder Milz, dienen kann. Die Verbindungen der Formel I akkumulieren nicht im retikulo-endothelialen System (RES). Insbesondere im Falle von fetten Ölen (Glyceriden) werden Transportvorgänge im vaskulären System, beispielsweise vom RES zur Lunge, gefördert.

Die Verbindungen der Formel I sind bifunktionell, aber nicht bipolar wirksam. Diese Eigenschaft bewirkt, daß diese Verbindungen entsprechend ihrer Umgebung micellare oder inverse micellare Strukturen ausbilden können oder - in Übereinstimmung mit dem CST-Wert - bei begrenzter oder vollständiger Mischbarkeit auch im Medium molekular gelöst sind.

Auf die große chemische Inertheit und mangelnde Toxizität wurde bereits hingewiesen. Die Verbindungen der Formel I sind jedoch auch nicht proliferativ: In Zelltests an Carcinoma-Zellen wird deren Proliferation durch Verbindungen der Formel I nicht beeinflußt.

Eine besondere Ausgestaltung der Erfindung betrifft die Verwendung der Emulsionen als Kalibriervngsflüssigkeiten für Blutgasanalysen. Die Partialdrücke von Sauerstoff und Kohlendioxid im Blut müssen häufig kontrolliert und kalibriert werden, um auftretende Fehlerabweichungen auszuschließen. Als Standards für die automatisierten Bestimmungen sind Referenzproben mit einem kleinen Volumen in einer verschlossenen Ampulle notwendig. Hierfür wird der pH-Wert der Emulsionen durch Zusatz eines Puffers zwischen 7 und 8, der Sauerstoffpartialdruck zwischen 50 und 500 mbar und der Kohlendioxidpartialdruck zwischen 10 und 90 mbar eingestellt.

Die erfindungsgemäßen Emulsionen eignen sich selbstverständlich auch für technische Einsatzzwecke, beispielsweise als inerte Gasträger oder auch hier als Medium für stabile Fluorcarbonradikale wie $.C_9F_{19}$ oder $.C_{10}F_{21}$. Diese Systeme mit Fluorcarbonradikalen können in Polymerisationsverfahren eingesetzt werden, beispielsweise für die Emulsionspolymerisation von Fluorpolymeren, wobei das Radikal als Initiator wirkt und außerdem die Endabsättigung der Polymermoleküle mit einem Perfluoralkylrest bewirkt.

In der folgenden Tabelle werden Verbindungen der Formel I mit wichtigen anwendungstechnischen Parametern aufgeführt. Aus diesen Daten ergibt sich die Eignung dieser Verbindungen für die erfindungsgemäßen Emulsionen.

In den folgenden Beispielen beziehen sich Prozentangaben auf das Gewicht.

## Tabelle

| Nummer | Verbindung (I) | MG [g/mol] | $O_2$-Löslichkeit [%, ml/ml] | CST [°C, n-Hexan] | Toxizität | |
| | | | | | $LD_{50}$ [g/kg] | Zelltest [%] [4] |
| --- | --- | --- | --- | --- | --- | --- |
| 1 | $C_8F_{17}H$ | 420 | 52,1 | 21 | | |
| 2 | $C_{10}F_{21}H$ | 520 | | 38 [3] | > 20 | |
| 3 | $C_6F_{13}C_2H_5$ | 348 | 46,1 | m [1] | | 97 |
| 4 | $C_8F_{17}C_2H_5$ | 448 | 45,6 | | > 20 | 96 |
| 5 | $C_{10}F_{21}C_2H_5$ | 548 | 43,4 | m | > 20 | 100 |
| 6 | $C_6F_{13}C_3H_7$ | 362 | 45,7 | m | | |
| 7 | $C_6F_{13}C_8H_{17}$ | 420 | 40,3 | m | | |
| 8 | $C_6F_{13}C_2H_4$—⟨H⟩ | 430 | 43,0 | m | | 93 |
| 9 | $C_6F_{13}C_2H_4$—[O,O] | 444 | | m | | |
| 10 | $C_6F_{13}OC_4H_9$ | 392 | 45,8 | m | | |
| 11 | $C_8F_{17}C_8H_{17}$ | 532 | 45,4 | m | | |
| 12 | $C_8F_{17}C_2H_4$—⟨H⟩ | 530 | 40,0 | m | | |
| 13 | $H-(C_8F_{17}CHCH_2)_4-H$ | 1790 | | 77 [3] | d [2] | |

1) m - mischbar ab -10 °C

2) d - dermatologisch getestet

3) experimentell bestimmt durch Extrapolation nach
J. Fluorine Chem. 61 (1993) 11

4) J. Fluorine Chem. 58 (1992) 201

Beispiel 1

30 g 1H-Perfluoroctan (Substanz 1) und 3,5 g Sojaöl werden bei Raumtemperatur intensiv gemischt und mit einer 9%igen wäßrigen Phospholipidlösung (PHOSPHOLIPON 80, Nattermann) zu einem Volumen von 50 ml ergänzt. Das ternäre Gemisch wird unter Eiskühlung mit einem Ultraschalldesintegrator hoher Energiedichte homogenisiert, bis die Teilchen einen mittleren Durchmesser von 110 nm aufweisen. Die Emulsion wird im Autoklav bei 121 °C sterilisiert. Nach Zusatz von Adjuvantien und Einstellung der Emulsion auf die physiologischen Bedingungen ist diese intravenös oder auch extrakorporal, zum Beispiel bei angioplastischen Verfahren als sauerstofftransportierendes Medium, anwendbar.

Beispiel 2

8 g Fluoroctylethylenoligomer (Substanz 13) und 3 g Kokosnußöl werden unter Rühren mit einer 5%igen wäßrigen Sojalecithin-Dispersion zu einem Volumen von 50 ml ergänzt. Durch nachfolgende Homogenisierung wird eine 16%ige Öl-in-Wasser-Emulsion mit Teilchengrößen von 120 nm erhalten. Diese wird mit dem gleichen Volumen einer Creme-Basis, bestehend aus Polyoxyethylenester, Glycerintriester, Cerylalkohol, Glycerin und Wasser, nach Standardverfahren in eine hautschonende Creme eingearbeitet. Die erhaltene Creme beziehungsweise Lotion bildet auf der Haut einen dünnen, stabilen, gasdurchlässigen Film und dient zum Schutz gegen irritierend wirkende Stoffe und zur Behandlung bei Kontakt-Dermatitis.

Beispiel 3

22,5 g F-Hexyl-n-H-propan (Substanz 6) und 2,5 g flüssiges Paraffin werden in einem Mixer gemischt und unter Zusatz von 20 ml einer 6%igen wäßrigen Lecithinlösung emulgiert. Es wird ein viskoses Gel erhalten, dem die Vitamine A und E, Aloe vera sowie Stabilisatoren und Konservierungsmittel zugesetzt werden. Das Gel ist auf der Haut zur Wundheilung, insbesondere von Verbrennungen, geeignet.

Beispiel 4

14 g 1H-Perfluordecan (Substanz 2) und 3 g flüssiges Paraffin werden bei 35 °C gemischt und mit 1,5 g eines Ethylenoxid-Propylenoxid-Blockpolymer-Emulgators (®PLURONIC F 37) und Wasser versetzt. Die Mischung mit einem Gesamtvolumen von 50 ml wird 8 Minuten bei 25 °C homogenisiert. Anschließend wird der Emulsion 1 ml eines flüssigen Fluorcarbongemisches, bestehend aus dem stabilen Fluorcarbonradikal $.C_{10}F_{21}$ und dessen Ausgangskomponente $C_{10}F_{20}$, zugesetzt. Nach einer weiteren Homogenisierung von 2 Minuten wird eine stabile, wenig viskose wäßrige Zubereitung erhalten. Die Messung des Radikals in einer Flachglasküvette mit einem ESR-Spektrometer im X-Band (9,5 GHz) ergibt ein gut aufgelöstes 10-Linienspektrum hoher Intensität. Bei Lagerung bei 6 °C ist das Radikal in der wäßrigen Dispersion länger als 1 Jahr ohne Intensitätsverlust haltbar. Die Probe kann als Spin-Marker angewendet werden.

Anstelle von Substanz 2 können auch die Substanzen 1, 3 und 6 verwendet werden. Der Emulgator wird durch ein Phospholipid (Sojalecithin) substituiert.

Beispiel 5

50 g des Perfluorhexyl-H-butylethers (Substanz 10) werden mit 5 g Paraffinöl vermischt und mit 5 g eines Ethylenoxid-Propylenoxid-Blockpolymer-Emulgators (PLURONIC F 68) und Wasser versetzt. Nach Hochdruckhomogenisierung bei 300 atm. wird eine stabile, 50%ig wäßrige viskose Emulsion erhalten, die eine $O_2$-Löslichkeit von 13 ml Sauerstoff/100 ml aufweist. Die Emulsion ist geeignet für die Übertragung von Sauerstoff in chemischen und technischen Systemen; bei Zusatz eines Puffersystems ist sie zur Kalibrierung von Blutgasanalysen anwendbar.

Anstelle des Ethers können auch die Substanzen 3 (40 %) und 6 (30 %) verwendet werden. Der Emulgator wird durch ein Fluortensid (2,5 %) ersetzt.

**Patentansprüche**

1. Emulgatorhaltige wäßrige Emulsionen von Ölen, enthaltend eine Verbindung der Formel I

   $(R_F)_x$-$R_H$     (I)

   in der $R_F$ einen hochfluorierten Alkylrest, der unmittelbar oder über ein Bindeglied an die Gruppe $R_H$ gebunden ist, $R_H$ einen Alkanrest oder Wasserstoff bedeutet und x für eine Zahl von 1 bis 4 steht.

2. Emulsionen nach Anspruch 1, enthaltend eine Verbindung der Formel I, in der $R_F$ über Sauerstoff oder Schwefel oder eine Gruppe der Formel II

   $-(O-CH_2-CH_2)_p-$     (II),

   in der p für 1 oder 2 steht, an $R_H$ gebunden ist, mit der Maßgabe, daß $R_H$ nicht Wasserstoff bedeutet, wenn $R_F$ über Sauerstoff oder Schwefel gebunden ist.

3. Emulsionen nach Anspruch 1 oder 2, enthaltend eine Verbindung der Formel I, in der $R_H$ einen Rest der Formel III

$$C_qH_{2q+1-x} \qquad (III)$$

bedeutet, in der x für eine Zahl von 1 bis 4 und q für eine ganze Zahl von 1 bis 12 steht.

4. Emulsionen nach Anspruch 1, enthaltend eine Verbindung der Formel IV

$$C_nF_{2n+1}\text{-}X\text{-}C_mH_{2m+1} \qquad (IV)$$

in der n 1 bis 20 bedeutet, wobei der Rest $C_nF_{2n+1}$ endverzweigt oder linear ist, X Sauerstoff, Schwefel oder eine Gruppe der Formel II

$$\text{-}(O\text{-}CH_2\text{-}CH_2)_p\text{-} \qquad (II)$$

bedeutet, in der p 1 oder 2 ist, und m eine Zahl von 0 bis 12 bedeutet, wenn X eine Gruppe der Formel II ist, oder eine Zahl von 1 bis 12 bedeutet, wenn X Sauerstoff oder Schwefel ist.

5. Emulsionen nach Anspruch 1, enthaltend ein Oligomer einer Verbindung der Formel V

$$Y\text{-}(CF_2)_a\text{-}O_b\text{-}(CH_2)_c\text{-}CH = CH_2 \qquad (V)$$

oder ein Co-Oligomer aus einer Verbindung der Formel V mit Verbindungen der Formel VI

$$Y\text{-}(CX_2)_d\text{-}O_b\text{-}(CX_2)_c\text{-}CX = CX_2 \qquad (VI)$$

in denen Y Wasserstoff oder Fluor, a eine Zahl von 2 bis 16, b und c - unabhängig voneinander - 0 oder 1 und d eine Zahl von 0 bis 6 bedeuten, mit einem mittleren Oligomerisationsgrad von 2 bis 4.

6. Emulsionen nach den Ansprüchen 1 bis 5, in denen als Öl nicht mit Wasser mischbare, bei Zimmertemperatur flüssige Verbindungen aus der Reihe der fetten Öle und der Kohlenwasserstofföle enthalten sind.

7. Verfahren zur Herstellung der Emulsionen nach Anspruch 1, dadurch gekennzeichnet, daß man eine Verbindung der Formel I mit einem Öl mischt und den wäßrigen Emulgator zusetzt.

8. Verfahren nach Anspruch 7, dadurch gekennzeichnet, daß die wäßrige Zubereitung des Emulgators absatzweise oder kontinuierlich unter Durchmischung zugesetzt wird und die so erhaltene Mischung anschließend einer Hochdruckhomogenisierung bei 100 bis 600 bar ausgesetzt wird.

9. Verwendung der Emulsion nach Anspruch 1 als Carrier von Wirkstoffen oder als Sauerstoffträger, insbesondere als sauerstofftransportierende Zubereitung auf der Haut.

10. Verwendung einer Emulsion nach Anspruch 1 als Standard in der Blutgaskontrolle.

Europäisches
Patentamt

**EUROPÄISCHER RECHERCHENBERICHT**

Nummer der Anmeldung

EP 95 10 2244

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,A | EP-A-0 545 174 (HOECHST AG) 9.Juni 1993 --- | | A61K9/00 |
| D,A | EP-A-0 231 091 (CHILDREN'S HOSPITAL MEDICAL CENTRE) 5.August 1987 --- | | |
| A | WO-A-89 00848 (CHEMEX PHARMACEUTICALS INC.) 9.Februar 1989 ----- | | |
| | | | **RECHERCHIERTE SACHGEBIETE (Int.Cl.6)** |
| | | | A61K |
| | Der vorliegende Recherchenbericht wurde für alle Patentansprüche erstellt | | |

| Recherchenort | Abschlußdatum der Recherche | Prüfer |
|---|---|---|
| DEN HAAG | 13.Juni 1995 | Klaver, T |